# EUROPEAN PATENT APPLICATION

(11) **EP 3 939 563 A1**
(43) Date of publication of application: **19.01.2022**
(21) Application number: 21397507.1
(22) Date of filing: 02.07.2021
(51) Int. Cl.: A61K 8/9789, A23L 2/08, A61K 8/41, A61K 8/92, A61Q 1/10, B01D 5/00, A61K 8/73, A61K 8/60

(54) **COSMETIC COMPOSITIONS COMPRISING NORDIC PLANT DERIVED INGREDIENTS AND A MILD NEUTRALIZING AGENT**

(30) Priority: 03.07.2020 FI 20205713
(71) Applicant: Lumene Oy, 02780 Espoo (FI)
(72) Inventor: ISOHANNI, Tiina, 02780 Espoo (FI); MÄKINEN, Päivi, 02780 Espoo (FI); PESONEN, Terhi, 02780 Espoo (FI); PALMUJOKI, Ingela, 02780 Espoo (FI); VÄNTTINEN, Kristofer, 02780 Espoo (FI); SAHRAMO, Elina, 02780 Espoo (FI)
(74) Representative: Laine IP Oy

(57) **Abstract**

According to an example aspect of the present invention, there is provided a cosmetic composition comprising dimethylglucamine, oil or extract from Nordic berries, at least one cosmetically acceptable solvent, and optionally other cosmetically acceptable ingredients.

## Description

### FIELD

The present invention relates to cosmetic compositions, which contain dimethylglucamine as a neutralizing agent, oil or extract from Nordic berries, at least one solvent and optionally one or more additional ingredients. The invention also relates to the use of the composition for the treatment or care of skin or keratin fibres, particularly for treatment of skin and for coloring of eye brows or eye lashes, for example in the form skin creams, gels, lotions and mascaras.

### BACKGROUND

In cosmetic industry there is a growing trend towards natural ingredients and compositions containing them. According to the definition provided in the REACH regulation, "natural" refers to naturally occurring substances as such, unprocessed or processed only by manual, mechanical or gravitational means, by dissolution in water, by flotation, by extraction with water, by steam distillation or heating solely to remove water, or which is extracted from air by any means.

According to a recent ISO standard for cosmetic products (ISO 16128, 2016/2017), natural ingredients are cosmetic ingredients obtained only from plants, animals, micro-organisms or minerals, including those obtained from these materials by physical processes, fermentation reactions occurring in nature and leading to molecules which occur in nature, and other procedures of preparation including traditional ones (e.g. extraction using solvents) without intentional chemical modification.

As regards coloring or toning of keratin fibres, particular attention is paid to health risks, since the chemicals used for coloring can result in a range of adverse effects, including temporary skin irritation and allergy. To ensure that coloring agents and dyes contain only safe substances, the European Union has adopted legislation (EC 1223/2009, Annex IV List of Colorants Allowed in Cosmetic Products) to restrict the use to only certain chemicals in coloring agents and dyes.

Also some neutralizing agents included in cosmetic compositions have been found to cause irritation of skin, while often also causing an unpleasant scent of the cosmetic composition. There is thus a growing need for cosmetic compositions, wherein the use of synthetic, irritative and possibly hazardous chemicals could be avoided to the extent possible. At the same time, the ingredients of such cosmetic compositions should preferably be biodegradable and also odourless, if possible.

Only few documents disclosing the use of dimethylglucamine in cosmetic compositions are available. For example, US 20150125415 A1 discloses a cosmetic composition suitable for treatment of hair, wherein the composition may contain N-methylglucamine. US 20180282664 A1 relates to detergents and cleaners based on longer chain fatty acid salts or fatty acid soaps, wherein said detergents and cleaners may also comprise dimethylglucamine.

The present invention aims at providing cosmetic compositions, wherein the proportion of natural/plant origin derived or natural (plant based) ingredients can be increased as high as possible, particularly to at least 90%, or even to 100%, without adverse effects on the properties of the cosmetic compositions as regards efficacy, structure and manufacture of the product. In particular, the invention aims at providing novel cosmetic compositions for treatment and care of skin and for coloring of keratin fibres, typically eye lashes and eye brows, wherein the proportion of natural ingredients, derived natural ingredients or derived organic or mineral ingredients is at least 90% by weight, and which provide a combination of a mild neutralizing agent and the beneficial properties of Nordic berry ingredients in the same cosmetic composition.

### SUMMARY OF THE INVENTION

The invention is defined by the features of the independent claims. Some specific embodiments are defined in the dependent claims.

The present invention is based on the concept of using dimethylglucamine as a neutralizing agent in cosmetic compositions, together with ingredients derived from Nordic berries.

According to a first aspect of the present invention, there is thus provided a cosmetic composition comprising dimethylglucamine as a neutralizing agent, at least one oil or extract from Nordic berries, at least one cosmetically acceptable solvent, and optionally other cosmetically acceptable ingredients, wherein said cosmetic composition comprises a proportion of natural or derived natural ingredients or both which is at least 90% by weight of the composition.

According to a second aspect of the present invention, there is provided the use of a cosmetic composition comprising dimethylglucamine as a neutralizing agent, at least one oil or extract from Nordic berries, at least one cosmetically acceptable solvent, and optionally other cosmetically acceptable ingredients, wherein said cosmetic composition comprises a proportion of natural or derived natural ingredients or both which is at least 90% by weight of the composition, for the treatment or care of skin or keratin fibres.

Embodiments of the invention comprise a cosmetic composition for treatment or care of skin, comprising dimethylglucamine as a neutralizing agent, at least one oil or extract from Nordic berries, at least one cosmetically acceptable solvent, and optionally other cosmetically acceptable ingredients, such as additives or auxiliary agents, wherein said cosmetic composition comprises a proportion of natural or derived natural ingredients or both which is at least 90% by weight of the composition and the composition is a skin care cream, emulsion, lotion or gel.

Still further embodiments of the invention comprise a cosmetic composition for treatment of keratin fibres, typically for coloring of eye lashes or eye brows, comprising dimethylglucamine as a neutralizing agent, at least one oil or extract from Nordic berries, at least one cosmetically acceptable solvent, and optionally other cosmetically acceptable ingredients, such as additives or auxiliary agents, together with at least one coloring agent, which may be a natural or synthetic coloring agent, wherein said cosmetic composition comprises a proportion of natural or derived natural ingredients or both which is at least 90% by weight of the composition.

Considerable advantages are obtained by the invention. First, the invention provides cosmetic compositions, wherein the proportion of natural ingredients, derived natural ingredients or natural organic/mineral ingredients is at least 90 % by weight, preferably at least 95 % and up to 100 % by weight.

Second, the invention provides cosmetic compositions having a natural, mild odour as compared to some traditional cosmetic compositions, which typically have a strong smell of chemicals without a high amount of perfume. Moreover, the neutralizing agent of the cosmetic compositions of the invention in addition to being odourless is also biodegradable. The natural ingredients and derived natural ingredients from Nordic berries are also biodegradable. The invention thus provides cosmetic compositions, such as creams, emulsions, lotions, gels and mascaras, wherein the majority of the ingredients of the composition may consist of natural ingredients or derived natural ingredients or both and wherein the neutralizing agents are biodegradable and odourless.

Third, the plant based oils and extracts from Nordic berries provide the cosmetic compositions of the invention with antioxidants, which are known active beneficial ingredients in cosmetic products.

It has also been found that by incorporating antioxidant-containing oils and extracts from Nordic berries in cosmetic compositions wherein amines, such as dimethylglucamine, are used as neutralizing agents, it is possible to reduce the formation of nitrosamines in the composition. Several oils and extracts from Nordic berries, spray-dried berry powders included, such as oils and extracts from cloudberry, lingonberry, sea buckthorn, blackcurrant, rosehip and bilberry, particularly from seeds thereof, are rich in antioxidants and vitamins, especially vitamins C and E. Further examples of vitamin-rich oils and extracts from Nordic plants are oat oil, which is rich in vitamin E, as well as spray-dried cloudberry powder, which is particularly rich in vitamin C. In the present compositions the antioxidative properties of the oils and extracts from Nordic berries effectively reduce the formation of potentially carcinogenic nitrosamines from amines.

Further features and advantages of the present technology will appear from the following description of some embodiments.

### EMBODIMENTS

Within this disclosure, the terms "natural origin derived", "natural derived", "derived natural" and "plant origin derived" are used synonymously, when referring to the ingredients of the cosmetic compositions. Also the terms "natural based ingredients", "plant-based ingredients" and "natural ingredients" have the same meaning in the present context.

In the present context, the terms "derived natural", "natural/plant origin derived" and "natural/plant derived" refer to substances that originate from plants but which may have been modified to fit for certain cosmetic purposes. According to ISO 16128-1, "derived natural ingredients" are cosmetic ingredients of greater than 50 % natural origin, by molecular weight, by renewable carbon content, or by any other relevant methods, obtained through defined chemical and/or biological processes with the intention of chemical modification. Examples of derived natural substances are shea butter ethyl esters.

In the present context, "natural/plant based" or "natural" refers to substances that are obtained directly from nature/plants or are only slightly modified, correspondingly to the definition of natural ingredients by ISO 16128-1. An example of natural ingredients is cetylstearyl alcohol.

International standard ISO 16128, which provides guidelines on definitions for natural and organic cosmetic ingredients, may be used for assessment of whether a certain cosmetic ingredient is a natural or derived natural cosmetic ingredient. However, animal-based ingredients are not used in the products within scope of the present patent, as the applicant is committed to vegan suitable products.

In the present context, the term "Nordic plants", "Nordic berries" or "Nordic wild berries" refers mainly to plants and berries naturally growing in the Nordic area without agriculture or gardening. However, to ensure diversity of Nordic nature, sources of natural plant extracts and oils may also be cultured. The natural ingredients may thus originate from organic farming methods or from wild harvesting in compliance with national legislation.

The Nordic area refers to a geographical region in Northern Europe and the North Atlantic. Preferably, the "Nordic plants" and "Nordic berries" referred herein include plants and berries growing in the Nordic countries, which by definition include Denmark, Finland, Iceland, Norway, and Sweden, as well as Greenland, the Faroe Islands, the Åland islands and Svalbard archipelagos.

Nordic berries include also parts of said berries, such as leaves, roots, branches, and twigs, more preferably fruits of said berries.

It has been found that by combining the use of dimethylglucamine as a neutralizing agent in neutralization requiring cosmetic compositions with ingredients obtained from Nordic berries it is possible to prepare cosmetic compositions, wherein the proportion of derived natural ingredients and/or natural ingredients is high, typically at least 90 % by weight of the composition. In particular, the skin care compositions according to the invention contain a proportion of natural or derived natural ingredients, which is at least 90 % by weight, even up to 100 % by weight. This is a high number compared to traditional skin care compositions, which generally contain natural or plant based ingredients only in minor amounts.

In particular, the cosmetic compositions according to the invention contain a combination of dimethylglucamine, at least one oil or extract from Nordic berries, and at least one cosmetically acceptable solvent.

Dimethylglucamine (N,N-dimethyl-d-glucamine or methylmeglumine, C₈H₁₉NO₅) is a sugar based, mild alkaline neutralizing agent. It is a renewable, biodegradable agent without any hazard classification. Due to its advantageous properties, such as good buffering ability, neutralizing effect, mildness and easy-to-use liquid form, dimethylglucamine has been suggested for use in hair colors, hair straighteners, vegetable fatty acid soaps, shaving creams, aqueous and hydro-alcoholic gels and sun care products. However, the use of dimethylglucamine in cosmetic compositions together with Nordic plant oil or extracts has not been disclosed or suggested.

As stated above, Nordic plants typically include Nordic berries including parts thereof. However, also some oleiferous plants and cereals, such as canola and oat (canola oil, oat oil) may be included.

Nordic berries include but are not limited to Nordic berries selected from the group consisting of lingonberry (*Vaccinium vitis-idaea),* bilberry *(Vaccinium myrtillus),* cloudberry (*Rubus chamaemorus*)*,* cranberry (*Vaccinium oxycoccus* / *Oxycoccus palustris*)*,* small cranberry (*Vaccinium microcarpum*)*,* bog bilberry (*Vaccinium uliginosum*)*,* raspberry (*Rubus idaeus*)*,* crowberry (*Empetrum nigrum*)*,* rowanberry (*Sorbus aucuparia, Sorbus fennica*)*,* sea buckthorn (*Hippophaë rhamnoides*)*,* blackcurrant *(Ribes nigrum),* redcurrant (*Ribes rubrum*)*,* white currant (*Ribes rubrum* f. *leucocarpum*)*,* greencurrant *(Ribes nigrum),* gooseberry (*Ribes uva-crispa*)*,* aronia berry (*Aronia mitschurinii*)*,* rose hip (*Rosa rugose, Rosa canina*)*,* saskatoon (*Amelanchier alnifolia*)*,* Arctic bramble (*Rubus arcticus*)*,* stone bramble (*Rubus saxatilis*), strawberry (*Fragaria x ananassa*)*,* blackberry (*Rubus fruticosus*)*,* bearberry (*Arctostaphylos uva-ursi*)*,* wild strawberry (*Fragaria vesca*) and juniper berry (*Juniperus communis*)*.*

In a preferred embodiment, the Nordic berries are selected from the group consisting of lingonberry (*Vaccinium vitis-idaea*)*,* bilberry (*Vaccinium myrtillus*)*,* cloudberry (*Rubus chamaemorus*)*,* cranberry (*Vaccinium oxycoccus* / *Oxycoccus palustris*) and sea buckthorn (*Hippophaë rhamnoides*)*.*

In another preferred embodiment, the Nordic berries are selected from the group consisting of cloudberry (*Rubus chamaemorus*)*,* sea buckthorn (*Hippophaë rhamnoides*)*,* blackcurrant (*Ribes nigrum*) and rose hip (*Rosa rugose, Rosa canina*)*.*

For cosmetic purposes, oils or extracts are typically obtained from plants by using supercritical fluid extraction, particularly supercritical carbon dioxide extraction, if only appropriate for the plant in question. Traditional solvent extraction methods are usually not applicable for the preparation of substances intended for cosmetic use, due to an insufficient degree of purity and the inclusion of solvent residues. However, "mild" oil extraction methods such as cold pressing are acceptable for cosmetic purposes.

For example, several berry or berry seed oils and extracts are commercially available. Typically, they are produced as follows: Berries or berry seeds are first dried, pressed as cakes and milled and then extracted with CO₂. Also frozen berry pulp may be used. From this extraction the separated berry oil or berry seed oil is recovered, subjected to after-treatment and packed. The berry or berry seed extract may be obtained by extracting the residue from the CO₂ extraction process by using a solvent different from CO₂. Alternatively, berry oils and berry seed oils and extracts may be obtained by cold-pressing.

It is possible to use for example water, ethanol, ethanol/water, acetone, butylene glycol, butylene glycol/water, glycerine or their mixtures as solvents in the extraction of the residual material. After the second extraction, any solids are separated from the extract. Before packaging the extract is subjected to microbial filtration. Microwave or ultrasonic extraction of Nordic plants and berries may also be used. Another possible extraction process comprises subcritical water extraction.

Spray-dried berry powders may be prepared simply by pressing the juice from berry fruit, filtering and mixing the juice with maltodextrin, and then spray-drying the mixture. A typical ratio is 30% of berry juice and 70% of maltodextrin by weight, but the proportion of berry juice may vary for example from 25 to 45% by weight of the composition, 55-75% by weight of the composition being maltodextrin.

In some embodiments, the cosmetic compositions of the invention may also contain aromatic oils or aromatic extracts from Nordic plants, preferably from Nordic berries. Aromatic oils are volatile and liquid aroma compounds, which are poorly soluble in water and have an odor, which makes them suitable for use in perfumery and food flavouring. However, non-aromatic oils and non-aromatic extracts from Nordic berries are preferred for the purposes of the present invention.

In cosmetic compositions for treatment and/or coloring of skin and keratin fibres, Nordic berry extracts and oils may provide the cosmetic composition with skin and keratin fibre conditioning properties. Moreover, plant origin derived ingredients with moisturizing properties may be added, such as xylitol from birch or oats. Also birch sap as such or in fermented form may be used as a moisturizing additive.

Oils and aromatic oils from Nordic plants and/or berries may also provide the cosmetic compositions with a pleasant scent.

In one embodiment of the invention, the proportion of natural ingredients from Nordic berries is at least 0.01%, preferably at least 0.1 % or at least 0.5%, more preferably at least 1-50% by weight of the composition.

In one embodiment of the invention, the combined proportion of natural and derived natural ingredients is at least 95% and up to 100 % by weight of the composition.

The amount of oils or extracts from Nordic berries in the cosmetic composition according to the invention varies depending on the particular cosmetic product in question. Typically, the amount of oils and extracts is 0.01- 50% by weight of the composition, preferably 0.01 to 10% by weight, or more preferably 0.01 to 5% by weight.

However, when berry waters are used as solvents as described below, the amount of extracts from Nordic plants may increase to at least 1 %, preferably at least 50 % and up to 99.5 % by weight of the composition.

The cosmetic compositions according to the invention contain an amount of dimethylglucamine, which is at least 0.1%, at least 0.5% or at least 1% by weight of the composition. Typically, the cosmetic compositions of the invention contain 0.1-10%, 0.5-10% or 1-15% dimethylglucamine by weight of the composition.

A further embodiment of the invention is a cosmetic composition, comprising or consisting essentially of: at least 0.5% by weight of dimethylglucamine as a neutralizing agent; at least 0.5% by weight of at least one oil or extract from Nordic berries; at least one cosmetically acceptable solvent; and optionally other cosmetically acceptable ingredients, and wherein the total amount of natural/plant derived or natural/plant based ingredients is at least 90 %, preferably at least 95% and up to 100 % by weight of the composition.

In an embodiment of the invention, the oil or extract is derived from cloudberry, lingonberry, cranberry, or bilberry, preferably from cloudberry, lingonberry, or bilberry. Oils and extracts naturally also include waxes obtained from said plants, such as berry fruit based waxes.

In embodiments of the invention, the composition may comprise also at least one other neutralizing agent, preferably selected from the group consisting of sodium hydroxide, potassium hydroxide, monoethylamine (MEA), aminomethyl propanol (AMP), and aminomethyl propanediol (AMPD). The amount of pH adjusting agent needed is based on target pH.

Examples of solvents include but are not limited to water, an aqueous evaporation residue or left-over from concentration or spray-drying of berry juice (berry water), and alcohols, wherein the alcohols are preferably selected from ethanol, propylene glycol, glycerol and other plant origin derived glycols. Preferred solvents are water, plant origin derived ingredients like ethanol and glycerol, and berry waters,

Berry waters refer herein to side streams of berry industry, in particular to aqueous evaporation residues or left-overs from concentration of berry juice. Typically, berry waters are obtained when raw berry juice obtained by pressing berries is concentrated, usually by evaporation. The vaporized water, that contains small amounts of berry substance, is condensated. Typically the condensated vaporized water is discharged as a waste stream. Another type of berry water obtained as a side stream of berry industry is aqueous evaporation residue or left-over from spray drying of concentrated berry juice to produce spray dried berry powder. The solvent and part of the berry substances are vaporized in the drying gas and condensated in the drying chamber. Both above disclosed berry waters may be included in the compositions of the invention, where they may be considered as extracts from Nordic plants, although they may also function as solvents.

In an embodiment of the invention the at least one cosmetically acceptable solvent is an aqueous evaporation residue from concentration or spray-drying of berry juice in an amount of 0.01-99.5% (w/w) of the total weight of the composition

An embodiment of the invention is a cosmetic composition for treatment and care of skin, wherein the composition also contains coloring agents or colorants selected from the group consisting of natural and synthetic colorants, preferably from natural colorants.

A further embodiment of the invention is a cosmetic composition for coloring of eye lashes and eye brows, wherein the composition also contains coloring agents or colorants selected from the group consisting of natural colorants and synthetic colorants.

Colorants include natural and synthetic dyes and colorants. Examples of natural colorants are plant-based dyes, such as vegetable dyes, blackcurrant, bilberry, and birch charcoal. Other natural dyes include certain minerals, such as iron oxide.

Preferred examples of keratin fibres are eye lashes and eye brows.

Typical examples of compositions for treatment and/or coloring of keratin fibres include mascaras for coloring of eye lashes as well as various compositions for coloring of eye brows, such as eyebrow markers and eyebrow waxes.

In an embodiment, the composition according to the invention is thus a mascara composition or an eyebrow wax.

In embodiments of the invention the composition may also contain surfactants or emulsifying agents.

Surfactants or emulsifying agents include anionic, amphoteric, and non-ionic surfactants. Examples of anionic surfactants include but are not limited to cetyl phosphate and stearic acid.

Examples of amphoteric surfactants include but are not limited to betaine derivatives. Examples of non-ionic surfactants include but are not limited to alkyl glucosides, fatty alkanolamides, laureth-12, and deceth-3.

Examples of thickeners or thickening agents include any thickening agent suitable in the field of cosmetics, as long as it is compatible with the other components of the composition and preferably a natural or derived natural ingredient, including glyceryl dibehenate, carbomers, vegetable derived gums like xanthan gum, acacia senegal gum and sclerotium gum, hectorites, glyceryl stearate, hydroxyethylcellulose, hydroxypropylmethylcellulose, Chondrus Crispus, magnesium stearate, magnesium silicate, aluminium silicate, magnesium aluminium silicate, cellulose derivatives and long chain alcohols. Thickening agents can be used alone or in combination with each other.

Usable waxes include natural waxes, such as bees wax, candelilla, carnauba, cereal based waxes, jojoba wax and their derivatives, berry fruit based waxes, birch based waxes (suberin), and sunflower seed wax.

Known keratin fibre conditioning agents include for example silicones, such as dimethicone and silanes, such as alkoxysilanes, cellulose derivatives, and fatty acid esters. However, also berry seed oils from Nordic berries as well Nordic plant extracts possess keratin fibre conditioning properties.

Silicones such as dimethicone, but also volatile silicon alternatives like ethyl oleate, C15-19 alkane, and isododecane and its natural origin alternatives like C13-15 alkane, may be used. Volatile silicones are preferably excluded.

In an embodiment of the invention, the composition of the invention comprises at least one other neutralizing agent in addition to dimethylglucamine. The at least one other neutralizing agent is preferably selected from the group consisting of sodium hydroxide, potassium hydroxide, monoethylamine (MEA), aminomethyl propanol (AMP), and aminomethyl propanediol (AMPD).

The composition according to the invention may also comprise other ingredients selected from the group consisting of other oils, waxes and fats, thickeners, preservative agents, perfume, chelating agents, pH adjusting agents, UV filters, opacifying agents, pearlescent agents, emulsifying agents, antioxidants, silicones, film forming polymers, humectants, fibres, emollients, vitamins, rheology modifiers and bulking agents like silica. Typically, the compositions of the present invention are free of longer chain fatty acid salts (fatty acid soaps) traditionally used in cosmetic compositions, in particular cleansing compositions.

Other oils, waxes, fats, resins, as well as pH adjusting agents, preservatives, antimicrobials and perfumes typically used in cosmetic products are known to a person skilled in the art.

Humectants include for example glycerol, betaine, xylitol, saccharide isomerate, birch sap and propylene glycol.

Examples of chelating agents are gluconates and EDTA, typically disodium-EDTA and naturally derived alternatives like sodium phytate or sodium gluconate.

All ingredients are selected to provide the cosmetic compositions of the invention with as high percentage proportion of natural /plant origin derived or natural ingredients as possible, without compromising other properties of the composition, such as skin caring effect, pleasant scent and structure of the composition.

In an embodiment of the invention, the composition according to the invention is used in mascaras. In mascara compositions, film forming polymers typically include ammonium acrylates copolymer, cellulose and corn derivatives and acetyl trihexyl citrate. Fibres include for example cellulose fibres while cetyl alcohol and various naturally derived waxes and oils are typically included as emollients.

In one embodiment, the coloring agent of a mascara composition is selected from the group of birch charcoal, iron oxide or both, wherein birch charcoal is preferred.

An embodiment of the invention is a mascara composition with a formula as referred in Example 1. Another embodiment of the invention is an eyebrow wax composition with a formula as disclosed in Example 3.

In an embodiment of the invention, the composition according to the invention is a skin care cream, lotion or gel. Typically, skin care compositions according to the invention comprise in addition to Nordic plant based ingredients also other cosmetically acceptable ingredients, such as oils (vegetable and/or mineral), waxes and fats, humectants, UV filters, emulsifying agents, silicones, thickeners, bulking agents, preservatives, and pH adjusting agents.

An embodiment of the invention is a skin care cream, lotion or gel composition with a formula as referred in Example 2.

It is to be understood that the embodiments of the invention disclosed are not limited to the particular structures, process steps, or materials disclosed herein, but are extended to equivalents thereof as would be recognized by those ordinarily skilled in the relevant arts. It should also be understood that terminology employed herein is used for the purpose of describing particular embodiments only and is not intended to be limiting.

Reference throughout this specification to one embodiment or an embodiment means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment. Where reference is made to a numerical value using a term such as, for example, about or substantially, the exact numerical value is also disclosed.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. In addition, various embodiments and example of the present invention may be referred to herein along with alternatives for the various components thereof. It is understood that such embodiments, examples, and alternatives are not to be construed as de facto equivalents of one another, but are to be considered as separate and autonomous representations of the present invention.

Furthermore, the described features, structures, or characteristics may be combined in any suitable manner in one or more embodiments. In the following description, numerous specific details are provided, such as examples of lengths, widths, shapes, etc., to provide a thorough understanding of embodiments of the invention. One skilled in the relevant art will recognize, however, that the invention can be practiced without one or more of the specific details, or with other methods, components, materials, etc. In other instances, well-known structures, materials, or operations are not shown or described in detail to avoid obscuring aspects of the invention.

### EXPERIMENTAL

### Example 1. Mascara composition

| **Ingredients** | **Maximum levels, % (w/w)** |
|---|---|
| Film forming polymers (e.g. ammonium acrylates copolymer, cellulose or corn based derivatives, acetyl trihexyl citrate) | 45 |
| Oils (e.g. vegetable and/or mineral), waxes and fats (e.g. long chain alcohols) | 30 |
| Colorants, colour additives (e.g. iron oxides, birch charcoal) | 25 |
| Emulsifying agents (e.g. stearic acid, neutralized palmitic acid) | 20 |
| Silicones (e.g. dimethicone) including volatile silicone alternatives (e.g. ethyl oleate, C15-19 alkane, and isododecane and its natural origin alternatives like C13-15 alkane) | 20 |
| Humectants (e.g. propylene glycol, glycerine, xylitol) | 15 |
| Thickeners (e.g. magnesium silicate, aluminium silicate, PEG derivatives, vegetable gums) | 15 |
| Ethanol (alcohol, alcohol denat.) | 11 |
| Fibres (e.g. cellulose) | 10 |
| Emollients (e.g. decyl oleate) | 5 |
| Additional ingredients (e.g. vitamins, Nordic berry seed oils, Nordic berry extracts, more precisely extracts from cloudberry, sea buckthorn, blackcurrant, rose hip, including spray-dried cloudberry powder and berry waters) | 15 |
| Preservatives, antimicrobials | 2.5 |
| pH adjusting agents (at least dimethylglucamine, other possible agents e.g. NaOH) | 0.1-15 |
| Perfume/No perfume | 0-1 |
| Aqua | to 100 |

### Example 2. Skin care cream, lotion or gel

| **Ingredients** | **Maximum levels, % (w/w)** |
|---|---|
| Oils (e.g. vegetable and/or mineral), waxes and fats (e.g. long chain alcohols | 60 |
| UV filters (both physical and chemical filters) | 30 |
| Humectants (e.g. glycerine, betaine, xylitol, saccharide isomerate) | 25 |
| Ethanol | 25 |
| Silicones (e.g. dimethicone) including volatile silicone alternatives (e.g. dimethicone ethyl oleate, C15-19 alkane, and isododecane and its natural origin alternatives like C13-15 alkane) | 20 |
| Emulsifying agents: anionic / amphoteric / non-ionic surfactants (e.g. cetyl phosphate, glyceryl stearate, PEG stearate) | 15 |
| Thickeners (e.g. carbomer, xanthan gum) | 12 |
| Bulking agents (e.g. talc, silica, cellulose powder) | 10 |
| Additional ingredients (e.g. antioxidants, vitamins, Nordic berry seed oils, Nordic berry extracts, more precisely extracts from cloudberry, sea buckthorn, blackcurrant, rose hip, including spray-dried cloudberry powder and berry waters) | 95 % |
| Preservatives, antimicrobials | 2 |
| Colorants | 1 |
| pH adjusting agents (at least dimethylglucamine) | 0.1-15 |
| Perfume/ Nordic berry/plant oil, aromatic oil | 1 |
| Aqua | to 100 |

### Example 3. Eyebrow wax composition

| **Ingredients** | **Maximum levels, % (w/w)** |
|---|---|
| Film forming polymers (e.g. ammonium acrylates copolymer, cellulose or corn based derivatives, acetyl trihexyl citrate) | 45 |
| Oils (e.g. vegetable and/or mineral), waxes and fats (e.g. long chain alcohols) | 30 |
| Colorants, colour additives (e.g. iron oxides, birch charcoal) | 25 |
| Emulsifying agents (e.g. stearic acid, neutralized palmitic acid) | 20 |
| Silicones (e.g. dimethicone) including volatile silicone alternatives (e.g. ethyl oleate, C15-19 alkane, and isododecane and its natural origin alternatives like C13-15 alkane) | 20 |
| Humectants (e.g. propylene glycol, glycerine, xylitol) | 15 |
| Thickeners (e.g. magnesium silicate, aluminium silicate, PEG derivatives, vegetable gums) | 15 |
| Ethanol (alcohol, alcohol denat.) | 11 |
| Fibres (e.g. cellulose) | 10 |
| Emollients (e.g. decyl oleate) | 10 |
| Additional ingredients (e.g. vitamins, Nordic berry seed oils, Nordic berry extracts, more precisely extracts from cloudberry, sea buckthorn, blackcurrant, rose hip, including spray dried cloudberry powder and berry waters) | 15 |
| Preservatives, antimicrobials | 2.5 |
| pH adjusting agents (at least dimethylglucamine, other possible agents e.g. NaOH) | 0.1-15 |
| Perfume/No perfume | 0-1 |
| Aqua | to 100 |

While the forgoing examples are illustrative of the principles of the present invention in one or more particular applications, it will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", that is, a singular form, throughout this document does not exclude a plurality.

As will be understood from the preceding description of the present invention and the illustrative experimental examples, the present invention can be described by reference to the following embodiments:
1. A cosmetic composition comprising:
   - dimethylglucamine as a neutralizing agent;
   - at least one oil or extract from Nordic plants;
   - at least one cosmetically acceptable solvent;
   and optionally other cosmetically acceptable ingredients.
2. The composition according to embodiment 1, wherein the oil or extract from Nordic plants comprises oil or extract from Nordic plants selected from the group of Nordic berries, trees, shrubs, subshrubs, flowers, and mushrooms, preferably from Nordic berries and trees.
3. The composition according to embodiment 1 or 2, wherein the Nordic berries are selected from the group consisting of lingonberry (*Vaccinium vitis-idaea*)*,* bilberry (*Vaccinium myrtillus),* cloudberry (*Rubus chamaemorus*)*,* cranberry (*Vaccinium oxycoccus* / *Oxycoccus palustris*)*,* small cranberry (*Vaccinium microcarpum*)*,* bog bilberry (*Vaccinium uliginosum*)*,* raspberry (*Rubus idaeus*)*,* crowberry (*Empetrum nigrum*)*,* rowanberry (*Sorbus aucuparia, Sorbus fennica*)*,* sea buckthorn (*Hippophaë rhamnoides*)*,* blackcurrant *(Ribes nigrum),* redcurrant (*Ribes rubrum*)*,* white currant (*Ribes rubrum* f. *leucocarpum*)*,greencurrant* (*Ribes nigrum*)*,* gooseberry (*Ribes uva-crispa*)*,* aronia berry (*Aronia mitschurinii*)*,* saskatoon (*Amelanchier alnifolia*)*,* Arctic bramble (*Rubus arcticus*)*,* stone bramble (*Rubus saxatilis*)*,* strawberry (*Fragaria x ananassa*)*,* blackberry (*Rubus fruticosus*)*,* bearberry (*Arctostaphylos uva-ursi*)*,* wild strawberry (*Fragaria vesca*) and juniper berry (*Juniperus communis*)*,* preferably from the group consisting of lingonberry, bilberry, cloudberry, cranberry and sea buckthorn.
4. The composition according to any one of the preceding embodiments, wherein the Nordic trees are selected from the group consisting of birch (*Betula*)*,* pine (*Pinus*), particularly Scots pine (*Pinus sylvestris*)*,* spruce (*Picea*), juniper (*Juniperus communis*)*,* aspen (*Populus tremula*)*,* alder (*Alnus*)*,* and willow (*Salix*).
5. The composition according to any one of embodiments 2 to 4, wherein the Nordic flowers comprise rosebay willoherb (fireweed, *Chamaenerion angustifolium*) and Marigold (*Calendula Officinalis*) and the Nordic subshrubs comprise heather (*Calluna vulgaris*).
6. The composition according to any one of embodiments 2 to 5, wherein the Nordic mushrooms are selected from the group consisting of chaga mushroom (*Inonotus obliquus*), bracket fungi or polypores (*Ganoderma*)*.*
7. The composition according to any one of the preceding embodiments, wherein the at least one cosmetically acceptable solvent is selected from the group consisting of water, an aqueous evaporation residue from concentration or spray-drying of berry juice, and alcohols, wherein the alcohols are preferably selected from ethanol, propylene glycol, glycerol and other plant origin derived glycols, more preferably the at least one solvent is water, ethanol, glycerol or an aqueous evaporation residue from concentration or spray-drying of berry juice.
8. The composition according to any one of the preceding embodiments, wherein the at least one cosmetically acceptable solvent is an aqueous evaporation residue from concentration or spray-drying of berry juice and wherein the amount of the aqueous evaporation residue is 0.01-99.5% (w/w) of the total weight of the composition.
9. The composition according to any one of the preceding embodiments for coloring of eye lashes or eye brows, wherein the composition further comprises at least one coloring agent selected from the group consisting of natural and synthetic colorants.
10. The composition according to embodiment 9, wherein the colorant is a natural colorant selected from the group consisting of birch charcoal, vegetable dyes, and iron oxide.
11. The composition according to embodiment 9, which is a mascara, wherein the coloring agent comprises birch charcoal, iron oxide or both.
12. The composition according to any one of embodiments 1 to 8, wherein the composition is a skin care cream, emulsion, lotion or gel.
13. The composition according to any one of the preceding embodiments, which comprises a proportion of natural ingredients, wherein the proportion of natural ingredients from Nordic plants is at least 0.01%, preferably at least 0.1% or at least 0.5%, more preferably at least 1-50% by weight of the composition.
14. The composition according to any one of the preceding embodiments, which comprises a proportion of natural or derived natural ingredients or both, wherein the proportion of natural or derived natural ingredients or both is at least 80%, preferably at least 90%. more preferably at least 95 %, by weight of the composition.
15. The composition according to any one of the preceding embodiments, wherein the proportion of oil or extract from Nordic plants is 0.01 - 50% by weight of the composition, preferably at least 0.01 to 10% by weight of the composition, typically 0.01 to 5% by weight of the composition.
16. The composition according to any one of the preceding embodiments, which contains dimethylglucamine in an amount of at least 0.1 %, at least 0.5 % or at least 1 % by weight of the composition, preferably 0.1-10%, more preferably 0.5-10% or 1-15%, by weight of the composition.
17. The composition according to any one of the preceding embodiments, comprising
   - at least 0.5% dimethylglucamine as a neutralizing agent;
   - at least 0.5% by weight of at least one oil or extract from Nordic plants;
   - at least one cosmetically acceptable solvent;
   and optionally other cosmetically acceptable ingredients, wherein the total amount of natural or derived natural ingredients or both is at least 80%, more preferably at least 90% by weight of the composition.
18. Use of the cosmetic composition according to any one of the preceding embodiments for the treatment or care of skin or keratin fibres, particularly for the treatment or care of skin, eye lashes and eye brows.

### INDUSTRIAL APPLICABILITY

At least some embodiments of the present invention find industrial application in cosmetic industry, by providing cosmetic compositions including mascaras, skin creams, skin lotions or skin gels.

### ACRONYMS LIST

- AMP: aminomethyl propanol
- AMPD: aminomethyl propanediol
- EDTA: ethylenediaminetetraacetic acid
- ISO: International Organization for Standardization
- MEA: monoethylamine
- PEG: polyethylene glycol
- REACH: registration, evaluation, authorization and restriction of chemicals
- TEA: triethanolamine

### CITATION LIST

### Patent Literature

US 20150125415 A1
US 20180282664 A1

### Non Patent Literature

EC 1223/2009, Annex IV, List of Colorants Allowed in Cosmetic Products
ISO 16128-1: Guidelines on technical definitions and criteria for natural and organic cosmetic ingredients and products - Part 1: Definitions for ingredients. 2016-02-15
ISO 16128-2: Cosmetics - Guidelines on technical definitions and criteria for natural and organic cosmetic ingredients - Part 2: Criteria for ingredients and products. 2017-09

## Claims

1. A cosmetic composition comprising:
- dimethylglucamine as a neutralizing agent;
- at least one oil or extract from Nordic berries;
- at least one cosmetically acceptable solvent;
- a proportion of natural or derived natural ingredients or both, wherein the proportion of natural or derived natural ingredients or both is at least 90% by weight of the composition;
and optionally other cosmetically acceptable ingredients.

2. The composition according to claim 1, wherein the Nordic berries are selected from the group consisting of lingonberry (*Vaccinium vitis-idaea*)*,* bilberry *(Vaccinium myrtillus),* cloudberry (*Rubus chamaemorus),* cranberry (*Vaccinium oxycoccus* / *Oxycoccus palustris*)*,* small cranberry (*Vaccinium microcarpum),* bog bilberry (*Vaccinium uliginosum*)*,* raspberry (*Rubus idaeus*)*,* crowberry (*Empetrum nigrum*)*,* rowanberry (*Sorbus aucuparia, Sorbus fennica*)*,* sea buckthorn (*Hippophaë rhamnoides*)*,* blackcurrant (*Ribes nigrum),* redcurrant (*Ribes rubrum*)*,* white currant (*Ribes rubrum* f. *leucocarpum),* greencurrant *(Ribes nigrum),* gooseberry (*Ribes uva-crispa*)*,* aronia berry (*Aronia mitschurinii),* saskatoon (*Amelanchier alnifolia*)*,* Arctic bramble (*Rubus arcticus*)*,* stone bramble (*Rubus saxatilis*)*,* strawberry (*Fragaria x ananassa*)*,* blackberry (*Rubus fruticosus*)*,* bearberry (*Arctostaphylos uva-ursi*)*,* wild strawberry (*Fragaria vesca*) and juniper berry (*Juniperus communis*)*,* preferably from the group consisting of lingonberry, bilberry, cloudberry, cranberry and sea buckthorn.

3. The composition according to claim 1 or 2, wherein the at least one cosmetically acceptable solvent is selected from the group consisting of water, an aqueous evaporation residue or left-over from concentration or spray-drying of berry juice, and alcohols, wherein the alcohols are preferably selected from ethanol, propylene glycol, glycerol and other plant origin derived glycols, more preferably the at least one solvent is water, ethanol, glycerol or an aqueous evaporation residue or left-over from concentration or spray-drying of berry juice.

4. The composition according to any one of the preceding claims, wherein the at least one cosmetically acceptable solvent is an aqueous evaporation residue or left-over from concentration or spray-drying of berry juice and wherein the amount of the aqueous evaporation residue is 0.01-99.5% (w/w) of the total weight of the composition.

5. The composition according to any one of the preceding claims for coloring of eye lashes or eye brows, wherein the composition further comprises at least one coloring agent selected from the group consisting of natural and synthetic colorants.

6. The composition according to claim 5, wherein the colorant is a natural colorant selected from the group consisting of birch charcoal, vegetable dyes, and iron oxide.

7. The composition according to claim 5, which is a mascara, wherein the coloring agent comprises birch charcoal, iron oxide or both.

8. The composition according to any one of claims 1 to 4, wherein the composition is a skin care cream, emulsion, lotion or gel.

9. The composition according to any one of the preceding claims, which comprises a proportion of natural ingredients, wherein the proportion of natural ingredients from Nordic berries is at least 0.01%, preferably at least 0.1% or at least 0.5%, more preferably at least 1-50% by weight of the composition.

10. The composition according to any one of the preceding claims, wherein the proportion of natural or derived natural ingredients or both is at least 95 % by weight of the composition.

11. The composition according to any one of the preceding claims, wherein the proportion of oil or extract from Nordic berries is 0.01 - 50% by weight of the composition, preferably at least 0.01 to 10% by weight of the composition, typically 0.01 to 5% by weight of the composition.

12. The composition according to claim 11, wherein the oil or extract from Nordic berries comprises or is spray-dried cloudberry powder.

13. The composition according to any one of the preceding claim, which contains dimethylglucamine in an amount of at least 0.1 %, at least 0.5 % or at least 1 % by weight of the composition, preferably 0.1-10%, more preferably 0.5-10% or 1-15%, by weight of the composition.

14. The composition according to any one of the preceding claims, comprising
- at least 0.5% dimethylglucamine as a neutralizing agent;
- at least 0.5% by weight of at least one oil or extract from Nordic berries;
- at least one cosmetically acceptable solvent;
and optionally other cosmetically acceptable ingredients, wherein the total amount of natural or derived natural ingredients or both is at least 90% by weight of the composition.

15. Use of the cosmetic composition according to any one of the preceding claims for the treatment or care of skin or keratin fibres, particularly for the treatment or care of skin, eye lashes and eye brows.
